# FASCICULE DE BREVET EUROPEEN

(11) **EP 0 609 425 B1**
(45) Date de publication et mention de la délivrance du brevet: **14.07.1999**
(21) Numéro de dépôt: 93919386.8
(22) Date de dépôt: 13.08.1993
(51) Int. Cl.: A61B 17/58

(54) **DISPOSITIF D'OSTEOSYNTHESE**
OSTEOSYNTHETISCHE VORRICHTUNG
OSTEOSYNTHESIS DEVICE

(30) Priorité: 25.08.1992 FR 9210247
(43) Date de publication de la demande: 10.08.1994
(73) Titulaire: WORCEL, Alexandre, F-75012 Paris (FR); KOVACS, Eric, F-93600 Aulnay-sous-Bois (FR)
(72) Inventeur: WORCEL, Alexandre, F-75012 Paris (FR); KOVACS, Eric, F-93600 Aulnay-sous-Bois (FR)
(74) Mandataire: Sueur, Yvette
(86) Numéro de dépôt international: FR9300811
(87) Numéro de publication internationale: WO9404086

(56) Documents cités:
- EP-A- 0 085 493
- EP-A- 0 340 413
- EP-A- 0 424 734
- EP-A- 0 468 600
- WO-A-88/03781
- DE-A- 3 725 111
- DE-A- 3 922 044
- FR-A- 2 674 119
- GB-A- 2 173 565
- US-A- 2 267 925
- US-A- 2 490 364

## Description

La présente invention concerne un dispositif destiné au maintien et à la compression des deux fragments d'un os fracturé.

On connaît de nombreux dispositifs destinés à maintenir en compression deux fragments d'un os fracturé. Ainsi, le brevet US 3 717 146 décrit un goujon intramédullaire de compression comprenant des extrémités coniques filetées que l'on introduit dans les fragments distal et proximal de l'os fracturé, et qui viennent en prise avec le tissu osseux. Le brevet US 4 091 806 décrit un clou de compression intramédullaire comportant une tête en forme de tige filetée qui dépasse à partir de l'extrémité de l'un des fragments. Cette tête s'étend à travers un manchon à extrémité libre en forme de fourche dont les branches sont écartées par un organe fileté vissé sur la tige à l'intérieur du manchon ; les branches s'enfoncent ainsi dans les parois du canal médullaire. Le brevet FR 2 459 650 décrit un dispositif intramédullaire de compression propre à être introduit dans une cavité creusée dans un os fracturé en deux parties, dispositif dont un élément comporte des organes élastiques s'étendant radialement dans la cavité et présentant un diamètre supérieur à celui de la cavité. De tels dispositifs, qui sont des dispositifs intramédullaires, ne sont pas utilisables pour des fractures de type épiphysaire. Le brevet US 3 990 438 décrit un dispositif de fixation et de compression pour os fracturés, qui comprend une première partie pourvue de filets coupants propres à s'ancrer dans le fragment principal de l'os et une deuxième partie filetée pour coopérer avec une vis qui s'étend à l'extérieur de l'os et qui est appliquee contre l'extrémité de celui-ci pour réaliser la compression axiale. Ce type de vis présente un double inconvénient : d'une part, la tête de la vis ne présente pas une surface suffisante pour coopérer de manière efficace avec le filetage de l'extrémité distale de la vis en vue d'assurer une bonne compression ; d'autre part, il se produit dans ce type de vis une incarcération osseuse entre les filets de la partie distale filetée qui rend très difficile l'ablation du la vis. Le brevet français 2 633 345 décrit une broche d'assemblage par ancrage constituée par une gaine munie de plusieurs corolles expansibles convergentes, coulissantes, comportant plusieurs pétales terminés en griffes ou en burins ; les corolles expansibles, en butée par leur ancrage dans la paroi intérieure d'un canal, agissant en rapprochement puis en compression sous l'effet d'une vis. Un tel système permet effectivement le maintien en compression d'un os fracturé. Toutefois, il est fixé suivant le principe du harpon, les corolles portant les griffes ou les burins étant convergentes ; dès lors que le dispositif est ancré dans l'os par les burins ou griffes écartés, l'extraction du dispositif en cas de nécessité ne peut se faire sans un traumatisme important. Compte tenu de l'incarcération osseuse qui se produit, il ne suffit pas de procéder à un dévissage pour dégager les griffes ou les burins et extraire la vis.

EP-A-0 340 413 décrit un dispositif pour consolider des fragments d'os, constitué par une cheville à expansion et une vis. La cheville est introduite dans une cavité forée dans l'os, ensuite, le vissage de la vis provoque le raccourcissement de la cheville et une augmentation de son diamètre, ce qui empêche la vis de sortir de l'os.

La présente invention a pour but de fournir un dispositif permettant le maintien en compression des deux parties d'un os fracturé, utilisable notamment pour des fractures épiphysaires, dispositif pouvant être installé et retiré si nécessaire avec un ancillaire classique sans dégradation supplémentaire de l'os.

A cet effet, la présente invention a pour objet un dispositif permettant le maintien en compression des deux parties d'un os fracturé, constitué par une vis dont la pointe est pyramidale ou conique et dont le corps est muni, à son extrémité distale, d'un filetage extérieur, caractérisé en ce que :
- le corps de la vis est cylindrique et creux ;
- la tête de la vis est constituée par des ailettes repliables solidaires de l'extrémité proximale du corps de la vis ;
- le dispositif comprend un élément cylindrique interne placé à l'intérieur du corps de la vis et susceptible de subir un déplacement axial par rapport au corps cylindrique de la vis, ledit élément cylindrique étant muni de moyens susceptibles d'agir sur l'extrémité libre des ailettes en vue de les déplier ou de les replier.

L'utilisation d'une vis selon la présente invention permet de réaliser une traction du corps de la vis, ce qui met la fracture en compression.

Dans un dispositif selon l'invention, les ailettes sont solidaires de l'extrémité proximale du corps de la vis. A l'état non monté, les ailettes s'étendent axialement. Lorsque le dispositif est mis en place dans un os, les ailettes sont repliées et forment une tête de vis pratiquement plate.

De préférence, le corps de la vis est un cylindre creux. Les moyens pour replier les ailettes peuvent être constitués par un élément cylindrique interne placé dans le corps de la vis et susceptible de subir à celui-ci, ledit élément interne étant muni de moyens susceptibles d'agir sur l'extrémité libre des ailettes.

Les moyens permettant de réaliser le déplacement de l'élément cylindrique dans le corps de la vis peuvent être constitués par un filetage sur la face interne du corps de la vis et par un filetage complémentaire sur la face externe de l'élément cylindrique interne.

Le dispositif de la présente invention est utilisable pour la mise en place de butées osseuses dans les opérations de reconstruction de hanche par exemple, et dans les butées d'épaule du type Latarjet. Dans la chirurgie du rachis, il présente en outre un grand intérêt pour le vissage pédiculaire, de l'odontoïde et dans le traitement chirurgical des spondylolysthésis.

Lorsque la nature de la fracture nécessite la mise en place d'une broche avant la mise en compression des fragments osseux, le dispositif de la présente invention comporte un canal central permettant le passage de la broche. Tel est le cas pour les fractures du scaphoïde, les fractures de la malléole interne, les butées de hanche ou d'épaule, les fractures de Garden 1 et 2 du col du fémur, les fractures pertrochantériennes du fémur, et de manière générale, pour les fractures des petits os.

L'élément cylindrique interne peut être un cylindre creux ou un cylindre plein.

Lorsque le dispositif de l'invention est destiné à prendre appui sur l'os spongieux, le filetage extérieur adjacent à la pointe de la vis est un filetage très large, dit filetage à spongieuse.

Lorsque le dispositif est destiné à prendre appui sur la corticale, le filetage extérieur est un filetage fin, dit filetage à corticale.

La présente invention est décrite plus en détail ci-dessous à l'aide des modes de réalisation particuliers, représentés sur les figures 1 à 10, donnés pour illustrer l'invention, qui n'est cependant pas limitée aux exemples donnés.
La figure 1 représente une vue en coupe axiale d'un dispositif selon l'invention, ailettes partiellement repliées.
La figure 2 représente une vue en coupe axiale d'un dispositif selon l'invention, ailettes repliées.
La figure 3 est une vue de la tête de la vis, avec des ailettes partiellement repliées.
La figure 4 est une vue en coupe de la figure 1 selon AA'.
La figure 5 est une vue en coupe selon BB'.
La figure 6 est une vue en coupe d'un dispositif selon l'invention, sans canal central.
La figure 7 est une vue agrandie de l'épaulement 16.
La figure 8 représente un dispositif comportant des ailettes distales avant mise en compression.
La figure 9 représente un dispositif comportant des ailettes distales après insertion dans un os et mise en compression.
La figure 10 représente une plaque vue du dessus.
avec un plaque.

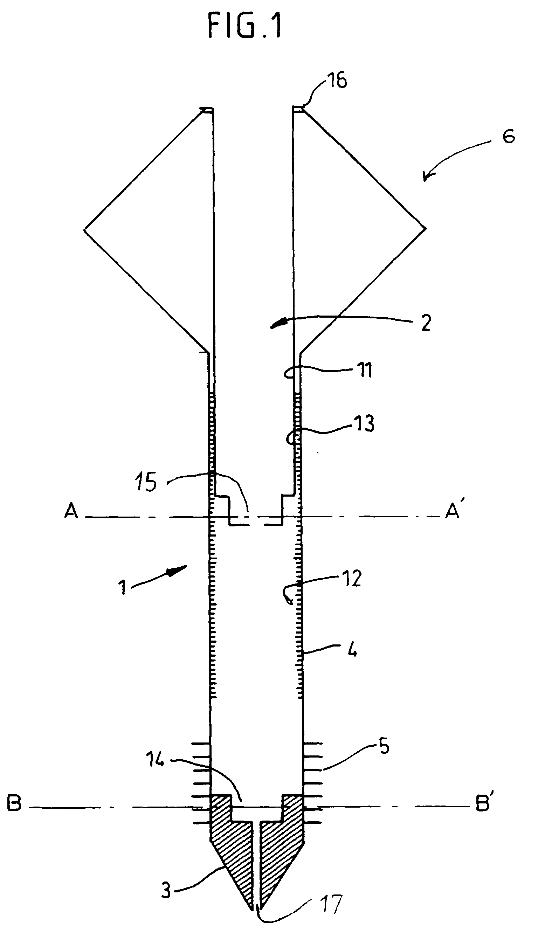

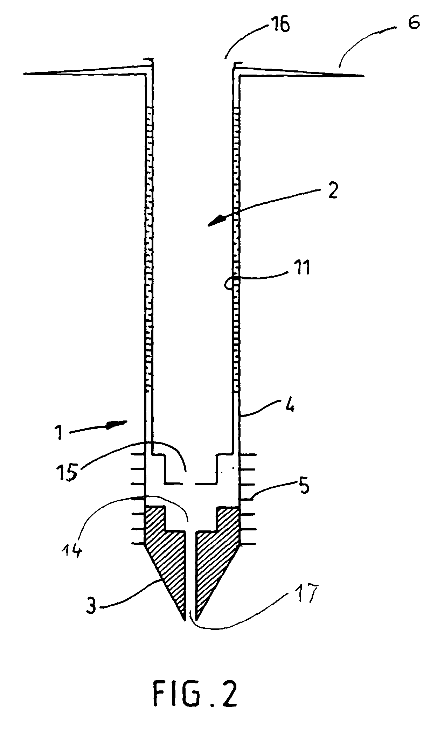

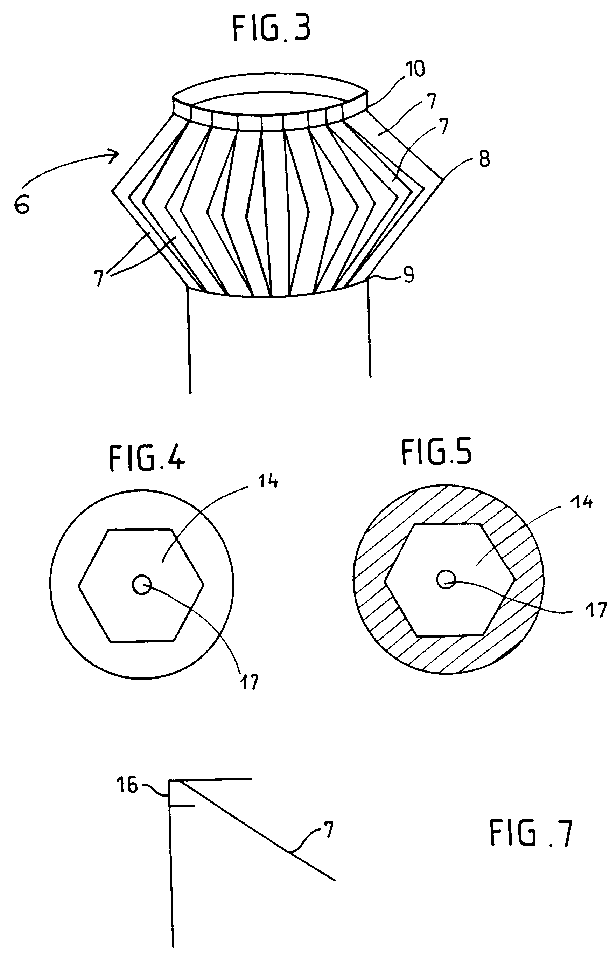

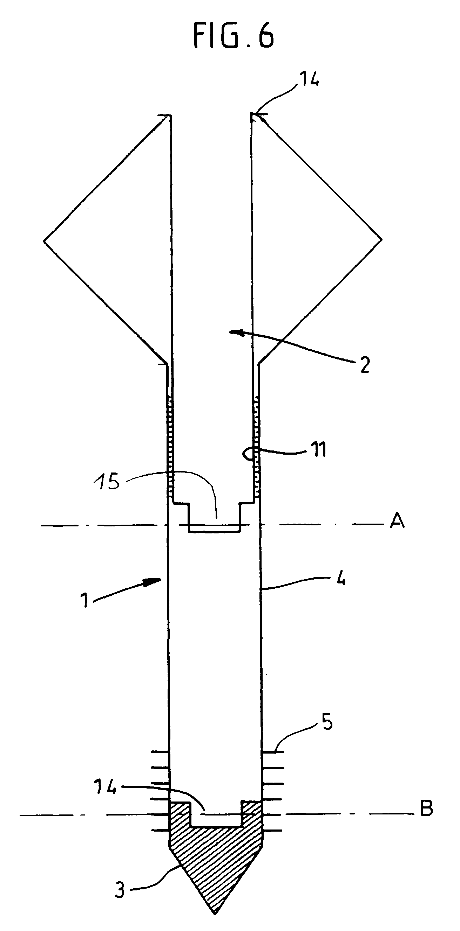

Le dispositif représenté sur les figures 1 et 2 est constitué par une vis 1 et un élément interne 2 et il comporte un canal central 17. La vis 1 comporte une pointe pyramidale 3, un corps 4 comportant un pas de vis externe 5 à son extrémité distale, et une tête 6 constituée par des ailettes 7. Le détail de la tête de la vis, ailettes partiellement repliées, est représenté sur la figure 3. Les ailettes 7 sont obtenues avantageusement par des incisions longitudinales dans la partie du corps 4 opposée à la pointe 3. Des pliures 8, 9 et 10 sont préformées pour obtenir un repliement des ailettes dans le sens souhaité. La vis 1 comporte en outre une empreinte 14 de forme polygonale permettant l'utilisation d'un tournevis pour insérer la vis dans la corticale de l'os. De préférence, l'empreinte est hexagonale (fig. 5), en vue de l'utilisation d'un tournevis à tête hexagonale classique.

L'élément interne 2 est constitué par un cylindre creux 11 comportant à l'une de ses extrémités un épaulement 16 sur lequel viennent buter les extrémités libres des ailettes 7.

La vis 1 et l'élément interne 2 sont munis de moyens permettant leur déplacement relatif axial. Dans le mode de réalisation représenté, ces moyens sont constitués par un pas de vis 12 sur la face interne de la vis 1 et un pas de vis complémentaire 13 sur la face externe de l'élément interne 2. Les pas de vis sont disposés de telle sorte que les ailettes soient allongées et que le vissage tendant à enfoncer l'élément interne dans le corps de la vis provoque le repliage des ailettes. L'élément interne 2 comporte une empreinte hexagonale 15 dans un plan radial, à son extrémité distale, en vue de l'utilisation d'un tournevis classique à tête hexagonale (fig. 4). Dans ce cas, l'élément interne est inséré dans le corps de la vis après que la vis soit mise en place dans l'os. L'ablation de l'élément interne 2 produit un dépliage des ailettes.

Dans une variante, dans laquelle on utilise un élément cylindrique interne creux, la face interne de l'élément interne 2 peut être munie des rainures longitudinales sur au moins une partie de sa longueur, ou de simples encoches qui permettent l'utilisation d'un tournevis cruciforme pour déplacer l'élément interne 2 par rapport à la vis 1.

Le dispositif représenté sur les figures 5 et 6 est un dispositif selon la présente invention sans canal central.

Le dispositif selon l'invention tel que décrit ci-dessus peut être mis en place pour maintenir en compression deux fragments d'un os fracturé à l'aide d'un ancillaire simple constitué par un tube qui comporte une extrémité distale dentée et un manche proximal antidérapant, et un tournevis à tête hexagonale.

Pour la pose d'un dispositif selon l'invention, la vis sélectionnée est mise en place dans le tube de telle sorte que seule sa pointe dépasse du tube, ensuite le tournevis est mis en place dans l'empreinte hexagonale de la vis et l'ensemble est posé sur la surface osseuse de telle sorte que les dents de l'extrémité distale se trouvent au contact de la surface osseuse. Le tournevis est provisoirement retiré.

On utilise avantageusement un tube comportant un encliquetage à ressort ou tout autre moyen pour éviter une chute de la vis.

Un impacteur peut être utilisé pour frapper sur le tube afin que les dents du tube se bloquent sur la corticale osseuse, empêchant ainsi le déplacement de l'ensemble sur la surface de la corticale. Après agrandissement du point d'entrée à l'aide d'une pointe carrée, le tournevis remis en place permet de perforer la corticale externe de l'os par des mouvements rotatoires alternatifs de la pointe de la vis. Lorsque la corticale est percée, la vis est enfoncée par vissage. La profondeur de pénétration de la vis doit être suffisante pour que le pas de vis externe 5 se trouve au-delà du trait de fracture et que la base des ailettes 7 soit en contact avec la surface de la corticale osseuse. On retire alors le tournevis hexagonal et le tube. Ensuite, on insère l'élément interne 2 dans le corps de la vis par vissage, soit à l'aide d'un tournevis cruciforme, soit à l'aide d'un tournevis à tête hexagonale, suivant la structure de cet élément interne. Cette opération provoque le repliement des ailettes et crée une compression par la coopération du pas de vis 5 et desdites ailettes 7.

Lorsque la nature de la fracture requiert la mise en place d'une broche, on utilise un dispositif selon l'invention comportant un canal central 17. Une réduction première de la fracture est stabilisée par une broche ayant une longueur adaptée à la nature de l'os fracturé. Un contrôle scopique de la position de la broche donne la dimension de la partie intra-osseuse de la broche et permet de choisir la taille de la vis à utiliser. Après agrandissement du point d'entrée a l'aide d'une pointe carrée, la vis sélectionnée est mise en place dans le tube de telle sorte que seule sa pointe dépasse du tube. Ensuite, un tournevis à tête hexagonale présentant un canal central est mis en place dans l'empreinte hexagonale de la vis. L'ensemble ainsi constitué est installé sur la broche, de telle sorte que les dents de l'extrémité distale du tube se trouvent au contact de la surface de la corticale osseuse. Le tournevis est provisoirement retiré.

Ensuite, on procède de la même manière que ci-dessus, mais en retirant la broche en même temps que le tube denté.

Dans le cas particulier de la fracture du scaphoide, l'élargissement du point d'entrée par une tarière ou une pointe carrée à main permet l'appui des ailettes 7 constituant la tête de la vis sur l'os spongieux et non plus sur la corticale osseuse, ce qui évite la saillie de la tête à la surface de l'os.

Dans le cas des vis à butée, du type Latarjet ou du type vis pour hanche par exemple, la broche centrale est facultative. La technique décrite ci-dessus facilite la pose d'une butée et permet même une réduction des dimensions de l'abord chirugical.

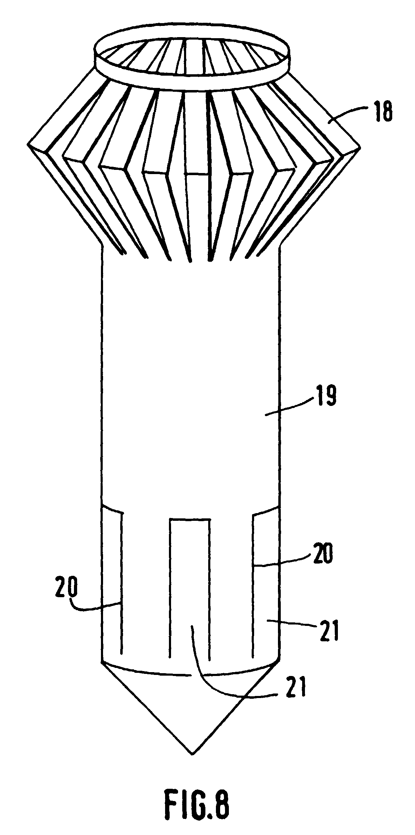

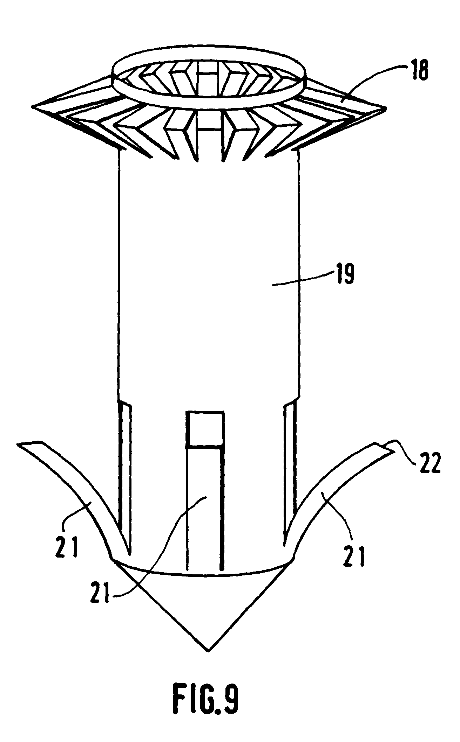

Les figures 8 et 9 représentent un mode de réalisation particulier du dispositif de la présente invention dans lequel la vis, comprenant une tête formée d'ailettes 18 et un corps 19, comporte en outre des ailettes 21 à sa partie distale, lesdites ailettes ayant une configuration telle qu'elles s'ouvrent en parapluie lorsque la partie distale de la vis est mise en traction par l'intermédiaire des ailettes repliables qui constituent la tête de la vis. Ces ailettes sont désignées par "ailettes distales". Les ailettes distales 21 peuvent être constituées par des incisions longitudinales 20 réalisées dans le corps 19 de la vis, lesdites incisions créant des lamelles qui restent solidaires du corps à leur extrémité la plus proche de la pointe, et qui peuvent s'écarter du corps à leur extrémité la plus proche de la tête, dite extrémité proximale 22. Les ailettes distales présentent un bord tranchant à leur extrémité proximale 22 de telle sorte que ces ailettes distales sont écartées du corps de la vis lorsque la vis est en traction, c'est-a-dire lorsque les ailettes qui constituent la tête de la vis sont repliées (fig.9). Dans ce mode de réalisation, le pas de vis extérieur situé à l'extrémité distale de la vis est du type à corticale et englobe les ailettes distales. De cette manière, le pas de vis n'accroche pas l'os lorsque la vis est mise en traction, ce qui va permettre l'ouverture des ailettes distales.

Bien entendu, une telle vis munie d'ailettes distales 21 peut comporter un canal central pour le passage d'une broche.

Une vis selon l'invention comportant des ailettes distales est particulièrement utile pour obtenir une bonne prise distale en diminuant le risque d'arrachement. En effet, son avantage réside dans la solidité du montage réalise et de l'effet de rappel elastique qui est créé et qui favorise la consolidation.

Une vis de la présente invention comportant des ailettes distales peut être posée à l'aide d'un ancillaire classique. Il s'agit d'un tube qui comporte une extrémité distale dentée qui permet de mieux bloquer le tube contre l'os, et un manche proximal antidérapant ; un tournevis éventuellement perforé pour le passage d'une broche, la tête du tournevis étant hexagonale et sa tige éventuellement graduée : eventuellement une broche ayant une longueur connue (en général de 13 mm) ; une mèche ayant un diamètre identique à celui de la vis ; un taraud présentant une butée, ayant un diamètre légèrement supérieur à celui de la vis, pour forer la corticale proximale de manière à permettre l'action de la tête à ailettes repliables de la vis ; et une règle plate graduée pour mesurer la partie de la broche qui dépasse de l'os.

Par exemple, pour la consolidation d'un pédicule, on repère le pédicule, puis on enfonce une broche prudemment jusqu'au contact de la corticale antérieure du corps vertébral, en effectuant un contrôle scopique. On mesure la partie de la broche qui dépasse, ce qui permet de déterminer la longueur de la vis à utiliser, connaissant la longueur totale de la broche. On positionne une mèche graduée sur la broche et on fore un trou d'une longueur supérieure de 3 mm à la longueur de la vis. On élargit la corticale proximale à l'aide d'une tarière, puis on positionne la vis munie de son tournevis dans le tube. On visse jusqu'à ce que le manche du tournevis soit en contact avec celui du tube. Après avoir retiré le tube, la broche et le tournevis, on visse l'élément cylindrique interne jusqu'à ce que les ailettes formant la tête de la vis soient complètement repliées, formant une tête plate et provoquant une traction sur la partie distale de la vis avec écartement concomitant des ailettes distales.

L'ablation du dispositif selon l'invention comportant ou non des ailettes distales, peut être effectuée sans que l'os soit endommagé. A cet effet, on utilise un ancillaire simple, à savoir le tournevis à tête hexagonale utilisé pour l'implantation de la vis, ou un tournevis comportant autant de petits crochets que la tête de vis comporte d'ailettes, ce qui permet de tirer sur les ailettes proximales tout en dévissant.

Pour certaines applications, un dispositif selon l'invention est utilisé en combinaison avec une plaque. Tel est le cas par exemple pour les fractures pertrochantériennes du fémur.

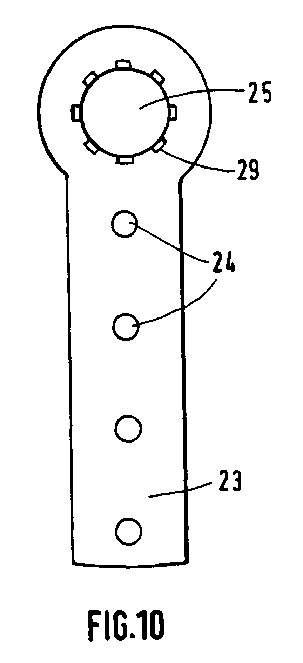

La fig. 10 représente une plaque connue en soi, vue du dessus.

La plaque 23 comporte des ouvertures 24 pour le passage de vis à corticale standard permettant de fixer la plaque à l'os, et un canon 25 permettant l'engagement du dispositif d'ostéosynthèse. L'orifice supérieur de la plaque 23 présente des encoches 29 dans lesquelles les ailettes 7 se bloquent, ce qui empêche la rotation de la vis.

L'utilisation d'une vis avec une plaque permet de combiner deux types de compression : d'une part, une compression par le poids du corps, la vis pouvant coulisser, par exemple sur 0,5 cm, avant d'être arrêtée par un encliquetage ; d'autre part une compression par la tête à compression. Le dispositif selon l'invention peut être utilisé avec une plaque sans qu'il soit nécessaire de prévoir de rails ou de méplats dans le canon de la plaque, ce qui permet de mieux le faire coulisser. La pose d'un ensemble vis-plaque comportant un dispositif selon la présente invention est grandement facilitée par le fait qu'il n'est plus nécessaire de terminer le vissage de la vis cervicale (lorsque le dispositif est utilisé pour une fracture du col du fémur ou d'une fracture pertrochantérienne par exemple), ce qui fait gagner au chirurgien au moins un quart d'heure, durée non négligeable pour le patient.

Le matériel ancillaire nécessaire pour installer un dispositif vis-plaque comprend un rapporteur oblique réglable de 120° à 140° ; deux tarières ayant des tailles appropriées, l'une servant à préparer le passage de la vis dans l'os, l'autre celui du canon de la plaque ; une broche, un tournevis à tête hexagonale et des tarauds de tailles successives.

Le dispositif vis-plaque selon l'invention est posé de la manière suivante, dans le cas d'une fracture du col du fémur ou d'une fracture pertrochantérienne. Après réduction de la fracture sous contrôle scopique, la broche est mise en place et sa position est vérifiée sous scopie. Le logement pour la vis est préparé par forage à la petite tarière jusqu'à 0,5 cm de l'os sous-chondral, puis à la grande tarière au niveau de la corticale externe. La plaque est mise en place sur son porte-plaque, puis positionnée sur le fémur. Ensuite la vis est vissée à l'aide d'un tournevis hexagonal, (éventuellement muni d'un système de blocage utilisant le pas de vis intérieur de la vis) dans le canon de la plaque jusqu'à ce que les ailettes se bloquent dans les encoches. Si la compression n'est pas souhaitée, le vissage est arrête a un repère dans le canon. Ensuite, la broche est retirée et l'élément cylindrique interne est vissé dans la vis pour provoquer le repliement des ailettes.

Les avantages du dispositif de l'invention sont nombreux. En premier lieu, il peut être installé sans qu'il soit nécessaire de creuser au préalable une cavité dans l'os à restaurer. Il peut être installé avec un ancillaire simple, et retiré si nécessaire avec le même ancillaire sans traumatisme ni lésion supplémentaire. Enfin, lorsque le dispositif est mis en place, et pendant tout le temps nécessaire à la restauration de l'os fracturé, aucun élément ne dépasse de la peau dans la mesure où la tête de la vis est plate.

## Revendications

1. Dispositif d'ostéosynthèse destiné à maintenir ensemble en compression les deux parties d'un os fracturé, constitué par une vis (1) dont la pointe (3) est pyramidale ou conique et dont le corps (4, 19) est muni, à son extrémité distale, d'un filetage extérieur (5), le corps (4, 19) de la vis (1) étant cylindrique et creux ; la tête (6) de la vis (1) étant constituée par des ailettes (7, 18) repliables solidaires de l'extrémité proximale du corps (4, 19) de la vis (1); le dispositif comprenant également un élément cylindrique interne (2) placé a l'intérieur du corps (4, 19) de la vis (1) et susceptible de subir un déplacement axial par rapport au corps (4, 19) cylindrique de la vis (1), ledit élément cylindrique (2) étant muni de moyens susceptibles d'agir sur l'extrémité libre des ailettes (7, 18) en vue de les déplier ou de les replier.

2. Dispositif selon la revendication 1, dans lequel les ailettes (7, 18) sont formées par des incisions axiales dans la partie cylindrique proximale du corps (4, 19) de la vis (1).

3. Dispositif selon la revendication 1, dans lequel les moyens permettant de réaliser le déplacement axial de l'élément cylindrique interne (2) dans le corps (4, 19) de la vis (1) sont constituées par un filetage (12) sur la face interne du corps (4, 19) de la vis (1) et par un filetage complémentaire (13) sur la face externe de l'élément cylindrique interne (2).

4. Dispositif selon la revendication 1, dans lequel l'élément cylindrique interne (2) comprend à l'une de ses extrémités un épaulement (16) sur lequel les extrémités libres des ailettes (7, 18) repliables sont susceptibles de buter.

5. Dispositif selon la revendication 1, dans lequel l'élément cylindrique interne (2) comporte un canal central (17) cylindrique présentant le même axe que le corps (4, 19) de la vis (1).

6. Dispositif selon la revendication 1, dans lequel le filetage extérieur (5) de l'extrémité distale de la vis (1) est un filetage large, dit filetage à spongieuse, ou un filetage fin, dit filetage à corticale.

7. Dispositif selon la revendication 1, dans lequel le corps (19) de la vis (1) comporte des ailettes distales (21) a son extrémité distale, et lesdites ailettes distales (21) s'étendent longitudinalement par rapport au corps (19) de la vis (1) et sont solidaires du corps (19) à leur extrémité distale.

8. Dispositif selon la revendication 1, associé à une plaque (23) munie d'un canon (25) dans lequel la vis (1) est insérée.

## Claims

1. An osteosynthesis device for holding together in compression two parts of a fractured bone, comprising a screw (1) the tip (3) of which is one of a pyramidal and conical shape and which has a body (4, 19) provided, at a distal end thereof, with an outside thread (5), wherein the body (4, 19) of the screw (1) is cylindrical and hollow, the head (6) of the screw (1) comprises foldable small wings (7, 18) integral with the proximal end of the body (4, 19) of the screw (1) ; said device further comprising an inner cylindrical element (2) located inside the body (4, 19) of the screw (1), which can undergo axial displacement in relation to the cylindrical body (4, 19) of the screw (1), said inner cylindrical element (2) being provided with means for acting on the free end of the small wings (7, 18) in view of folding or unfolding them.

2. A device according to claim 1, wherein the small wings (7, 18) are formed by making axial incisions in the proximal cylindrical part of the body (4, 19) of the screw (1).

3. A device according to claim 1, wherein the means for causing the inner cylindrical element (2) to undergo axial displacement in the body (4, 19) of the screw (1) comprises threads (12) located on the inner face of the body (4, 19) of the screw (1) and complementary threads (13) located on the outer face of the inner cylindrical element (2).

4. A device according to claim 1, wherein said inner cylindrical element (2) comprises at an end thereof, a flange (16) against which the free ends of the small foldable wings (7, 18) may strike.

5. A device according to claim 1, wherein the inner cylindrical element (2) comprises a cylindrical central channel (17) which has an axis corresponding to that of the body (4, 19) of the screw (1).

6. A device according to claim 1, wherein the outside thread pitch (5) of the distal end of the screw (1) comprises a wide pitch, so called spongy thread pitch, or a fine pitch, so called cortex thread pitch.

7. A device according to claim 1, wherein the body (19) of the screw (1) comprises distal small wings (21) at its distal end, and said distal small Wings (21) extend longitudinally in relation to the body (19) of the screw (1) and are integral with the body (19) at their distal end.

8. A device according to claim 1, which is combined with a plate (23) provided with a barrel (25) in which the screw (1) is inserted.

## Patentansprüche

1. Osteosynthesevorrichtung, die dazu bestimmt ist, die beiden Teile eines frakturierten Knochens aneinandergepreßt zu halten, bestehend aus einer Schraube (1), deren Spitze (3) pyramidenförmig oder konisch ist und deren Körper (4, 19) an seinem distalen Ende mit einem Außengewinde (5) versehen ist,
wobei der Körper (4, 19) der Schraube (1) zylindrisch und hohl ist;
wobei der Kopf (6) der Schraube (1) aus faltbaren Fahnen (7, 18) besteht, die einstückig mit dem proximlalen Ende des Körpers (4, 19) der Schraube (1) verbunden sind;
wobei die Vorrichtung auch ein zylindrisches Innenelement (2) umfaßt, das im Inneren des Körpers (4, 19) der Schraube (1) angeordnet ist und axiale Verlagerung in bezug auf den zylindrischen Körper (4, 19) der Schraube (1) erfahren kann, wobei das zylindrische Element (2) mit Mitteln versehen ist, die auf das freie Ende der Fahnen (7, 18) wirken können, um sie zu entfalten oder zu falten.

2. Vorrichtung nach Anspruch 1, worin die Fahnen (7, 19) durch axiale Einschnitte im zylindrischen proximalen Abschnitt des Körpers (4, 19) der Schraube (1) gebildet sind.

3. Vorrichtung nach Anspruch 1, worin die Mittel, die die axiale Verlagerung des zylindrischen Innenelements (2) im Körper der Schraube (1) ermöglichen, aus einem Gewinde (12) an der Innenfläche des Körpers (4, 19) der Schraube (1) und aus einem komplementären Gewinde (13) an der Außenfläche des zylindrischen Innenelements (2) bestehen.

4. Vorrichtung nach Anspruch 1, worin das zylindrische Innenelement (2) an einem seiner Enden einen Ansatz (16) umfaßt, an den die freien Enden der faltbaren Fahnen (7, 18) anstoßen können.

5. Vorrichtung nach Anspruch 1, worin das zylindrische Innenelement (2) einen zylindrischen Mittelkanal (17) umfaßt, der die gleiche Achse aufweist wie der Körper (4, 19) der Schraube (1).

6. Vorrichtung nach Anspruch 1, worin das Außengewinde (5) des distalen Endes der Schraube (1) ein großes, sogenanntes Spongiosa- Gewinde oder ein feines, sogenanntes Kortikalis- Gewinde ist.

7. Vorrichtung nach Anspruch 1, worin der Körper (19) der Schraube (1)distale Fahnen (21) an seinem distalen Ende umfaßt und die distalen Fahnen (21) in bezug auf den Körper (19) der Schraube (1) in Längsrichtung erstrecken und an ihrem distalen Ende einstückig mit dem Körper (19) verbunden sind.

8. Vorrichtung nach Anspruch 1, die einer Platte (23) zugeordnet ist, die mit einem Auge (25) versehen ist, in das die Schraube (1) eingesetzt wird.
